# EUROPEAN PATENT APPLICATION

(11) **EP 1 806 409 A1**
(43) Date of publication of application: **11.07.2007**
(21) Application number: 06025149.3
(22) Date of filing: 05.12.2006
(51) Int. Cl.: C12P 13/22, C12N 1/20

(54) **Method for producing aromatic L-Amino acid using bacterium belonging to the Genus Methylophilus**

(30) Priority: 27.12.2005 RU 2005140649
(71) Applicant: Ajinomoto Co., Inc., Tokyo 104-8315 (JP)
(72) Inventor: Tokmakova, Irina L'vovna, Istrinskiy district Moscow region 143514 (RU); Gorshkova, Natalya Vasilievna, Moscow 117454 (RU); Abalakina, Elena Georgievna, Moscow 123273 (RU); Iomantas, Yurgis Antanas Vladovich, Moscow 123273 (RU)
(74) Representative: Strehl Schübel-Hopf & Partner

(57) **Abstract**

The present invention provides a method for producing an L-amino acid using a bacterium belonging to the genus *Methylophilus* which has been modified to enhance expression of genes involved in the efflux of aromatic L-amino acids, particularly the *yddG, yddL, yddK* and *yddJ* genes from *Escherichia coli.*

## Description

### Background of the Invention

### Field of Invention

The present invention relates to the microbiological industry, and specifically to a method for producing an aromatic L-amino acid, particularly L-phenylalanine, using a bacterium belonging to the genus *Methylophilus,* wherein said bacterium has been modified to enhance expression of genes encoding proteins which are involved in aromatic L-amino acid efflux.

### Description of the Related Art

L-Amino acids such as L-lysine, L-glutamic acid, L-threonine, L-leucine, L-isoleucine, L-valine, and L-phenylalanine are industrially produced by fermentation using microorganisms that belong to the genus *Brevibacterium, Corynebacterium, Bacillus, Escherichia, Streptomyces, Pseudomonas, Arthrobacter, Serratia, Penicillium, Candida,* and the like. In order to improve the productivity, strains isolated from nature or artificial mutants thereof have been used. Various techniques have been disclosed to increase the L-glutamic acid-producing ability by enhancing activities of L-glutamic acid biosynthetic enzymes by using recombinant DNA techniques.

The production of L-amino acids has been considerably increased by breeding microorganisms such as those mentioned above and improving production methods. However, in order to meet increased demand in the future, development of more efficient methods for producing L-amino acids at a lower cost are still desirable.

To produce amino acids by fermentation of methanol, which is a raw material available in large amounts at a low cost, conventionally known methods include using microorganisms that belong to the genus *Achromobacter* or *Pseudomonas* (Japanese Patent Publication (Kokoku) No. 45-25273/1970), *Protaminobacter* (Japanese Patent Application Laid-open (Kokai) No. 49-125590/1974), *Protaminobacter* or *Methanomonas* (Japanese Patent Application Laid-open (Kokai) No. 50-25790/1975), *Microcyclus* (Japanese Patent Application Laid-open (Kokai) No. 52-18886/1977), *Methylobacillus* (Japanese Patent Application Laid-open (Kokai) No. 4-91793/1992), *Bacillus* (Japanese Patent Application Laid-open (Kokai) No. 3-505284/1991), and so forth.

A method for producing L-phenylalanine by culturing a bacterium belonging to the genus *Methylophilus* which has an ability to produce L-phenylalanine and is resistant to a phenylalanine analog, such as DL-p-fluorophenylalanine, m-fluorophenylalanine and cinnamic acid, has been disclosed (EP1134283B1).

Also, a method for producing an L-amino acid, including L-phenylalanine, by culturing a microorganism having an ability to produce an L-amino acid in a medium, whereby the L-amino acid accumulates in the medium, and collecting the L-amino acid from the medium, whereby said microorganism is a methanol-utilizing bacterium having the Entner-Doudoroff pathway in which 6-phosphogluconate dehydratase activity and/or 2-keto-3-dexoy-6-phosphogluconate aldolase activity is enhanced, has been disclosed (US patent application 20040142435A1).

A method for producing an L-amino acid, including L-phenylalanine, by culturing a microorganism which has been modified so that expression of an *ybjE* gene is enhanced in a liquid medium containing methanol as a major carbon source has also been disclosed (PCT application WO05073390A2).

A process for producing an L-amino acid, for example L-phenylalanine or L-tryptophan, by culturing an *Escherichia* bacterium in a medium, the L-amino acid productivity of the bacterium has been enhanced by elevating the activity of a protein encoded by the *yddG* gene only has been disclosed (PCT application WO03044192A1).

But currently, there have been no reports of enhancing expression of the *yddG, yddL, yddK* and *yddJ* genes for the purpose of producing L-amino acids, such as L-phenylalanine, using methanol-utilizing bacteria belonging to the genus *Methylophilus.*

### Disclosure of the Invention

Objects of the present invention include enhancing the productivity of aromatic L-amino acid-producing strains and providing a method for producing an aromatic L-amino acid using these strains.

The above objects were achieved by finding that enhancing expression of the *yddG, yddL, yddK* and *yddJ* genes from *E. coli* in a bacterium belonging to the genus *Methylophilus,* particularly *Methylophilus methylotrophus,* can increase production of aromatic L-amino acids, such as L-phenylalanine, L-tryptophan, and L-tyrosine, preferably L-phenylalanine.

The present invention provides a bacterium belonging to the genus *Methylophilus* having an increased ability to produce aromatic L-amino acids, such as L-phenylalanine, L-tryptophan, and L-tyrosine, preferably L-phenylalanine.

It is an object of the present invention to provide a bacterium belonging to the genus *Methylophilus* having an ability to produce an aromatic L-amino acid, wherein said bacterium has been modified to enhance expression of genes coding for proteins involved in aromatic L-amino acid efflux.

It is a further object of the present invention to provide the bacterium as described above, wherein said genes are obtained from a bacterium belonging to the genus *Escherichia.*

It is a further object of the present invention to provide the bacterium as described above, wherein said genes comprise *yddG, yddL, yddK* and *yddJ.*

It is a further object of the present invention to provide the bacterium as described above, wherein said expression is enhanced by modifying an expression control sequence which controls expression of said genes.

It is a further object of the present invention to provide the bacterium as described above, wherein said expression is enhanced by increasing the copy number of said genes.

It is a further object of the present invention to provide the bacterium as described above, wherein said aromatic L-amino acid is selected from the group consisting of L-phenylalanine, L-tyrosine, and L-tryptophan.

It is a further object of the present invention to provide the bacterium as described above, wherein said aromatic L-amino acid is L-phenylalanine.

It is a further object of the present invention to provide the bacterium as described above, wherein said bacterium is *Methylophilus methylotrophus.*

It is a further object of the present invention to provide a method for producing an aromatic L-amino acid comprising:
- cultivating the bacterium described above in a medium containing methanol as a major carbon source to produce and excrete said aromatic L-amino acid into the medium, and
- collecting said L-amino acid from the medium.

It is a further object of the present invention to provide the method as described above, wherein said aromatic L-amino acid is selected from the group consisting of L-phenylalanine, L-tyrosine, and L-tryptophan.

It is a further object of the present invention to provide the method as described above, wherein said aromatic L-amino acid is L-phenylalanine.

The present invention is described in detail below.

### Detailed Description of the Preferred Embodiments

### 1. Bacterium of the present invention

The bacterium of the present invention is a bacterium belonging to the genus *Methylophilus* having an ability to produce the aromatic L-amino acid, wherein said bacterium has been modified to enhance expression of genes involved in aromatic L-amino acid efflux.

In the present invention, "bacterium having an ability to produce an aromatic L-amino acid" means a bacterium which has an ability to produce and excrete L-amino acid into a medium when the bacterium is cultured in the medium. The term "bacterium having ability to produce aromatic L-amino acid" as used herein also means a bacterium which is able to produce and cause accumulation of an L-amino acid in a culture medium in an amount larger than a wild-type or parental strain of *M. methylotrophus,* such as *M. methylotrophus* AS1, and preferably means that the microorganism is able to cause accumulation in a medium of an amount not less than 0.05 g/L, more preferably not less than 0.1 g/L, of the target L-amino acid. The term "aromatic L-amino acid" includes L-phenylalanine, L-tryptophan, and L-tyrosine. L-phenylalanine is particularly preferred.

The genus *Methylophilus* includes the following bacteria: *Methylophilus freyburgensis, Methylophilus leisingeri, Methylophilus methylotrophus, Methylophilus quaylei* etc. Specifically, those classified as *Methylophilus* according to the taxonomy used in the NCBI (National Center for Biotechnology Information) database can be used and are included. A bacterium belonging to the species *Methylophilus methylotrophus* is preferred. Specific examples of *Methylophilus* bacteria which may be used in the present invention include the *Methylophilus methylotrophus* strain AS1 (NCIMB10515) and so forth. The *Methylophilus methylotrophus* strain AS1 (NCIMB10515) is available from the National Collections of Industrial and Marine Bacteria (Address NCIMB Lts., Torry Research Station, 135, Abbey Road, Aberdeen AB98DG, United Kingdom).

Genes coding for proteins involved in aromatic L-amino acid efflux include genes coding for proteins capable of pumping aromatic L-amino acids out of the bacterial cells. Increased activity of such proteins imparts resistance to aromatic L-amino acids of the bacteria and results in increasing accumulation of aromatic L-amino acids in the medium when such bacterium is cultivated in the medium.

The phrase "a bacterium belonging to the genus *Escherichia"* means that the bacterium is classified into the genus *Escherichia* according to the classification known to a person skilled in the art of microbiology. Examples of a bacterium belonging to the genus *Escherichia* as used in the present invention include, but are not limited to, *Escherichia coli* (*E. coli*).

The bacterium belonging to the genus *Escherichia* that can be used in the present invention is not particularly limited; however, e.g., bacteria described by Neidhardt, F.C. et al. (Escherichia coli and Salmonella typhimurium, American Society for Microbiology, Washington D.C., 1208, Table 1) are encompassed by the present invention.

The phrase "bacterium has been modified to enhance expression of genes" means that the bacterium has been modified in such a way that the modified bacterium contains increased amounts of the proteins coded by said genes, as compared with an unmodified bacterium.

The phrase "the enhanced expression of genes" means that the expression level of genes is higher than that of a non-modified strain, for example, a wild-type strain. Examples of such modifications include increasing the copy number of the expressed gene(s) per cell, and/or increasing the expression level of the gene(s) by modification of an adjacent region of the gene, including sequences controlling gene expression, such as promoters, enhancers, attenuators, ribosome-binding sites, etc., and so forth.

The phrase "genes coding for proteins involved in aromatic L-amino acid efflux" means that when culturing a microorganism which has been modified to enhance expression of the genes, the amount of L-amino acid secreted into the medium by the microorganism is more than that of an L-amino acid secreted from a non-modified strain, such as a parent strain or a corresponding wild-type strain. The increase in L-amino acid-efflux ability is observed by determining the increase in concentration of the L-amino acid in the medium. Furthermore, the increase in L-amino acid-efflux ability is also observed by determining the decrease in intracellular concentration of the L-amino acid upon introduction of the gene into a microorganism. The amount of L-amino acid secreted from the microorganism of the present invention is preferably increased by 10% or more, more preferably 30% or more, particularly preferably 50% or more, when compared to the amount of L-amino acid exported from a non-modified strain. Furthermore, the increase in L-amino acid-efflux ability is also observed in terms of a decrease in intracellular concentration of the L-amino acid upon introduction of genes into a microorganism. For example, the intracellular concentration of an L-amino acid can be measured as follows: an appropriate amount of silicon oil having specific gravity of 1.07 is added to a medium containing microbial cells, and cells are collected from the medium by centrifugation, preferably at 12,000rpm for 2 minutes. Then the cells are treated with 22% perchloric acid (Ishizaki, A. et al, Biotech.Techniq.,9, 6, 409 (1995)). Using thus prepared cells, an intracellular concentration of an L-amino acid can be measured. Furthermore, "L-amino acid-efflux ability" can be examined indirectly by measuring cellular uptake of radiolabeled L-amino acid using everted membrane vesicles (Pittman, M.S. et al, J. Biol. Chem., 277, 51, 49841-49849 (2002)). For example, everted membrane vesicles are prepared from cells into which gene is introduced. Then, ATP or other substrates which provide driving energy are added to the vesicles, and cellular uptake of radiolabeled L-amino acid is measured. Alternatively, "L-amino acid-export ability" may be examined by measuring the rate of the exchange reaction between a non-labeled amino acid and a labeled amino acid in active cells.

The examples of genes from bacterium belonging to the genus *Escherichia* involved in aromatic L-amino acid efflux include the *yddG, yddL, yddK* and *yddJ* genes from *E. coli.*

The *yddG* gene encodes the putative transmembrane YddG protein (synonym - B1473). The *yddG* gene (nucleotides complementary to nucleotides positions 1544312 to 1545193; GenBank accession no. NC_000913.2; gi:49175990; SEQ ID NO: 1) is located between the *yddL* and *fdnG* genes on the chromosome of *E. coli* K-12. The nucleotide sequence of the *yddG* gene and the amino acid sequence of the YddG protein encoded by the *yddG* gene are shown in SEQ ID NO: 1 and SEQ ID NO: 2, respectively.

The *yddL* gene encodes putative outer membrane porin YddL protein (synonym - B1472). The *yddL* gene (nucleotides complementary to nucleotides positions 1543762 to 1544052; GenBank accession no. NC_000913.2; gi:49175990; SEQ ID NO: 3) is located between the *yddG* and *yddk* genes on the chromosome of *E. coli* K-12. The nucleotide sequence of the *yddL* gene and the amino acid sequence of the YddL protein encoded by the *yddL* gene are shown in SEQ ID NO: 3 and SEQ ID NO: 4, respectively.

The *yddK* gene encodes the putative glycoprotein YddK (synonym - B 1471). The *yddK* gene (nucleotides complementary to nucleotides positions 1542782 to 1543738; GenBank accession no. NC_000913.2; gi:49175990; SEQ ID NO: 5) is located between the *yddJ* and *yddL* genes on the chromosome of *E. coli* K-12. The nucleotide sequence of the *yddK* gene and the amino acid sequence of the YddK protein encoded by the *yddK* gene are shown in SEQ ID NO: 5 and SEQ ID NO: 6, respectively.

The *yddJ* gene encodes the YddJ protein (synonym - B1470), the function of which is unknown. The *yddJ* gene (nucleotides complementary to nucleotides positions 1542408 to 1542743; GenBank accession no. NC_000913.2; gi:49175990; SEQ ID NO: 7) is located between the *narU* and *yddK* genes on the chromosome of *E. coli* K-12. The nucleotide sequence of the *fucU* gene and the amino acid sequence of the FucU protein encoded by the *fucU* gene are shown in SEQ ID NO: 7 and SEQ ID NO: 8, respectively.

Since there may be some differences in DNA sequences between *Escherichia* strains, the above-described genes to be overexpressed are not limited to the nucleotide sequences shown in SEQ ID NOS: 1, 3, 5 and 7, but may also include nucleotide sequences similar to those shown in SEQ ID NOS: 1, 3, 5 and 7. Therefore, the protein variants encoded by the above-described genes may have a similarity of not less than 80%, preferably not less than 90%, and most preferably not less than 95%, with respect to the entire amino acid sequences shown in SEQ ID NOS. 2, 4, 6 and 8, as long as the ability of the proteins to cause efflux of aromatic L-amino acids is maintained.

Moreover, the above-described genes may be represented by variants which can hybridize under stringent conditions with the nucleotide sequences shown in SEQ ID NOS: 1, 3, 5 and 7 or with probes prepared based on these nucleotide sequences, provided that they encode functional proteins. "Stringent conditions" include those under which a specific hybrid is formed and a non-specific hybrid is not formed. For example, stringent conditions are exemplified by washing at 60°C one time, preferably two or three times, with a solution containing 1 × SSC and 0.1% SDS, preferably 0.1 × SSC and 0.1% SDS. The length of the probe may be suitably selected, depending on the hybridization conditions, and usually varies from 100 bp to 1 kbp.

Enhancement of gene expression may be achieved by increasing copy number of the genes, and/or by introducing the vector carring the gene. The vector for *Methylophilus* bacteria, for example, is a plasmid that is autonomously replicable in cells of *Methylophilus* bacteria. Specific examples of vectors for *Methylophilus* bacteria include RSF1010 and derivatives thereof, such as pAYC32 (Chistorerdov, A.Y., Tsygankov, Y.D. Plasmid, 16,161-167 (1986)), pMFY42 (Gene, 44, 53 (1990)), pRP301, and pTB70 (Nature, 287, 396, (1980)).

Enhancement of gene expression may be achieved by introducing multiple copies of the gene into a bacterial chromosome by, for example, the method of homologous recombination, Mu integration, or the like. The copy number of an expressed gene can be estimated, for example, by restricting the chromosomal DNA followed by Southern blotting using a probe based on the gene sequence, by fluorescence *in situ* hybridization (FISH), and the like.

Enhancement of gene expression may also be achieved by placing the gene(s) under the control of a potent promoter suitable for functioning in the bacterium belonging to the genus *Methylophilus.* For example, the lac promoter, the *trp* promoter, the *trc* promoter, the P_{R}, or the P_{L} promoters of lambda phage are known as potent promoters. Use of a potent promoter can be combined with multiplication of gene copies.

Alternatively, the effect of a promoter can be enhanced by, for example, introducing a mutation into the promoter to increase the transcription level of a gene located downstream of the promoter. Furthermore, it is known that substitution of several nucleotides in the spacer region between the ribosome binding site (RBS) and the start codon, especially the sequences immediately upstream of the start codon, can profoundly affect the mRNA translatability. For example, a 20-fold range in the expression levels was found, depending on the nature of the three nucleotides preceding the start codon (Gold et al., Annu. Rev. Microbiol., 35, 365-403, 1981; Hui et al., EMBO J., 3, 623-629, 1984). Previously, it was shown that the *rhtA23* mutation is an A-for-G substitution at the -1 position relative to the ATG start codon (ABSTRACTS of 17th International Congress of Biochemistry and Molecular Biology in conjugation with 1997 Annual Meeting of the American Society for Biochemistry and Molecular Biology, San Francisco, California August 24-29, 1997, abstract No. 457). Therefore, it may be suggested that the *rhtA23* mutation enhances *rhtA* gene expression and, as a consequence, increases resistance to threonine, homoserine, and some other substances transported out of cells.

The level of gene expression can be estimated by measuring the amount of mRNA transcribed from the gene using various well-known methods, including Northern blotting, quantitative RT-PCR, and the like. The amount of the protein encoded by the gene can be measured by well-known methods, including SDS-PAGE followed by immunoblotting assay (Western blotting analysis) and the like.

Methods for preparation of plasmid DNA, digestion and ligation of DNA, transformation, selection of an oligonucleotide as a primer, and the like may be ordinary methods well-known to those skilled in the art. These methods are described, for instance, in Sambrook, J., Fritsch, E.F., and Maniatis, T., "Molecular Cloning: A Laboratory Manual, Second Edition", Cold Spring Harbor Laboratory Press (1989)

### L-amino acid-producing bacteria

The bacterium of the present invention can be obtained by enhancing expression of genes coding for proteins involved in aromatic L-amino acid efflux in a bacterium, which inherently has the ability to produce aromatic L-amino acids. Alternatively, the bacterium of present invention can be obtained by imparting the ability to produce aromatic L-amino acids to a bacterium already having the enhanced expression of genes coding for proteins involved in aromatic L-amino acid efflux.

The ability to produce aromatic amino acids may be an original ability of a wild-type strain of bacterium or an ability which has been imparted by breeding.

Hereinafter, methods for imparting an aromatic amino acid-producing ability to a parent strain as mentioned above will be explained.

In order to impart aromatic amino acid-producing ability, methods conventionally used for breeding an L-amino acid-producing bacterium belonging to the genus *Escherichia* or Coryneform bacterium and so forth can be used. For example, methods for obtaining an auxotrophic mutant strain, analogue-resistant strain, or metabolic regulation mutant strain having L-amino acid-producing ability, and methods for creating a recombinant strain having enhanced activity of an L-amino acid-biosynthetic enzyme can be used ("Amino Acid Fermentation", the Japan Scientific Societies Press [Gakkai Shuppan Center], 1st Edition, published on May 30, 1986, pp.77-100). When breeding L-amino acid-producing bacteria using these methods, one or more properties, including auxotrophy, analogue resistance, and metabolic regulation mutation, may be imparted.

When a recombinant strain is created, the activity of single or multiple L-amino acid-biosynthetic enzymes may be enhanced. Furthermore, methods imparting properties of auxotrophy, analogue resistance, and metabolic regulation mutation may be combined with methods enhancing an activity of L-amino acid-biosynthetic enzyme.

An auxotrophic mutant strain, L-amino acid analogue-resistant strain, or metabolic regulation-mutated strain having an L-amino acid-producing ability can be obtained by subjecting a parent or wild-type strain to a typical mutagenesis treatment such as X-ray or ultraviolet ray irradiation, treatment with a mutagenesis agent such as N-methyl-N'-nitro-N-nitrosoguanidine (NTG). Then, an auxotrophic strain, analogue-resistant strain or metabolic regulation mutant strain which has an L-amino acid-producing ability may be selected from the mutated strains.

Bacteria having an ability to produce aromatic amino acids can be obtained by enhancing the activities of 3-deoxy-D-arabinoheptulosonate-7-phosphate synthase (encoded by *aroG* gene), 3-dehydroquinate synthase (encoded by *aroB* gene), shikimate kinase (encoded by *aroL* gene), chorismate mutase (encoded by *aroA* gene), chorismate synthase (encoded by *aroC* gene) (PCT application WO02/38777).

### 2. Method of the present invention

The method of the present invention is a method for producing an L-amino acid by cultivating the bacterium of the present invention in a culture medium to produce and excrete the L-amino acid into the medium, and collecting the L-amino acid from the medium.

In the present invention, the cultivation, collection, and purification of L-amino acid from the medium and the like may be performed in a manner similar to conventional fermentation methods wherein an amino acid is produced using a bacterium.

A medium used for culture may be either a synthetic or natural medium, so long as the medium includes a carbon source and a nitrogen source and minerals and, if necessary, appropriate amounts of nutrients which the bacterium requires for growth. The carbon source for bacterium belonging to the genus *Methylophilus* includes C1 compounds, such as methanol. If methanol is used as the main carbon source, aromatic L-amino acids, such as L-phenylalanine, can be produced at low cost. When methanol is used as the main carbon source, it is added to a medium in an amount of 0.001 to 30%. As a nitrogen source, various ammonium salts such as ammonia and ammonium sulfate, other nitrogen compounds such as amines, a natural nitrogen source such as peptone, soybean-hydrolysate, and digested fermentative microorganisms can be used. As minerals, potassium monophosphate, magnesium sulfate, sodium chloride, ferrous sulfate, manganese sulfate, calcium chloride, and the like can be used. As vitamins, thiamine, yeast extract, and the like, can be used.

The cultivation is preferably performed under aerobic conditions, such as a shaking culture, and a stirring culture with aeration, at the temperature of 20 to 40°C, preferably 30 to 38°C. The pH of the culture is usually between 5 and 9, preferably between 6.5 and 7.2. The pH of the culture can be adjusted with ammonia, calcium carbonate, various acids, various bases, and buffers. Usually, 1 to 5-day cultivation leads to accumulation of the target L-amino acid in the liquid medium.

After cultivation, solids, such as cells, can be removed from the liquid medium by centrifugation or membrane filtration, and then the L-amino acid can be collected and purified by ion-exchange, concentration, and/or crystallization methods.

### Brief Description of Drawings

Figure 1 shows the structure of PCR-amplified DNA fragment carrying RBS^{fmd} and the *yddG, yddL, yddK, yddJ* genes from *E. coli* encoding a complete set for the phenylalanine efflux pump.
Figure 2 shows the structure of deletion derivatives carrying the *yddG* gene.

### Examples

The present invention will be more concretely explained below with reference to the following non-limiting Examples.

### Example 1. The cloning, analysis, and application of the L-phenylalanine efflux pump genes from E. coli in M. methylotrophus strains.

It has been reported that enhancing the activity of a protein encoded by the *yddG* gene from *E. coli* increases production of L-amino acid, for example L-phenylalanine or L-tryptophan, by *E. coli* bacterium when the bacterium is cultured in a medium (PCT application WO03044192A1).

Therefore, it was supposed that the *yddG* gene encodes an L-phenylalanine efflux pump.

The *M methylotrophus* is a good test microorganism for analysis of L-phenylalanine efflux genes, because it did not grow on medium containing high L-phenylalanine concentration (2.0 - 5.0 g/L). And the active L-phenylalanine efflux pump imparts resistance to phenylalanine to a *M. methylotrophus* bacterium.

At first, a single *yddG* gene from *E. coli* was amplified by PCR. It was amplified with new ribosome binding site, AGGAGA, of *M. methylotrophus* formamidase (*fmd*) gene (Mills, J. et al, Eur. J. Biochem., 251, p. 45-53 (1998)) using two synthetic primers P1 (SEQ ID NO: 9) and P2 (SEQ ID NO: 10). Primer P1 contains a *Sma*I restriction site and RBS of *M. methylotrophus* formamidase gene. Primer P2 contains a *Kpn*I restriction site. The chromosomal DNA of *E. coli* strain MG1655 was used as a template.

The resulting amplified DNA fragment was treated with *Sma*I and *Kpn*I restictases and ligated under the control of the P_{tac} promoter into the pTACTER2 vector which had been previously treated with the same restricases. The resulting plasmid was designated as pTYD6 (pTACTER2-Ptac^{Eco}-RBS^{fmd}-yddG^{Eco}).

The pTACTER2 vector was obtained from the pAYCTER3 vector by introducing a P_{tac} promoter. For that purpose, the commercially available P_{tac} promoter (Pharmacia, Sweden) was treated with *Hind*III and *Bam*HI restrictases and ligated into a pAYCTER3 vector which had been previously treated with the same restricases.

The pAYCTER3 vector is a derivative of pAYC32, a moderate copy number and very stable vector constructed on the basis of plasmid RSF1010 (Christoserdov A. Y., Tsygankov Y. D, Broad-host range vectors derived from a RSF 1010 Tnl plasmid, Plasmid, 1986, v. 16, pp. 161-167). The pAYCTER3 vector was obtained by introduction of the polylinker from the pUC19 plasmid and the strong terminator *rrnB* into the pAYC32 plasmid instead of its native terminator. Detailed contruction of pAYCTER3 vector is described in the (PCT application WO03044192A1).

Then, the resulting pTYD6 plasmid was mobilized from *E. coli* strain S17-1 (McPheat, W.L., FEMS Microbiology Letters, 41, 185-188 (1987)) into *M. methylotrophus* strain AS1. *Methylophilus methylotropus* strain AS1 is available from National Collections of Industrial and Marine Bacteria (Address NCIMB Lts., Torry Research Station, 135, Abbey Road, Aberdeen AB98DG, United Kingdom).

Both strains AS1 and the resulting AS1/pTYD6 were tested by fermenting in tubes in 121 medium with 2% methanol (conditions for fermentation and method for measurement of L-phenylalanine concentration: see Example 2 below). The fermentation data are presented in Table 1. It can be seen, the wild-type *M. methylotrophus* strain AS1 did not produce phenylalanine, when only the *yddG* gene from *E. coli* was amplified on the plasmid.

It was supposed that a single *yddG* gene from *E. coli* encodes only an inner membrane protein which is insufficient for functioning as an exporter across the inner and outer membranes in the *M. methylotrophus* strain. Thus, in order to form a multicomponent phenylalanine efflux pump in *M. methylotrophus* bacteria, it was necessary to find and clone other genes from *E. coli* besides the *yddG* gene.

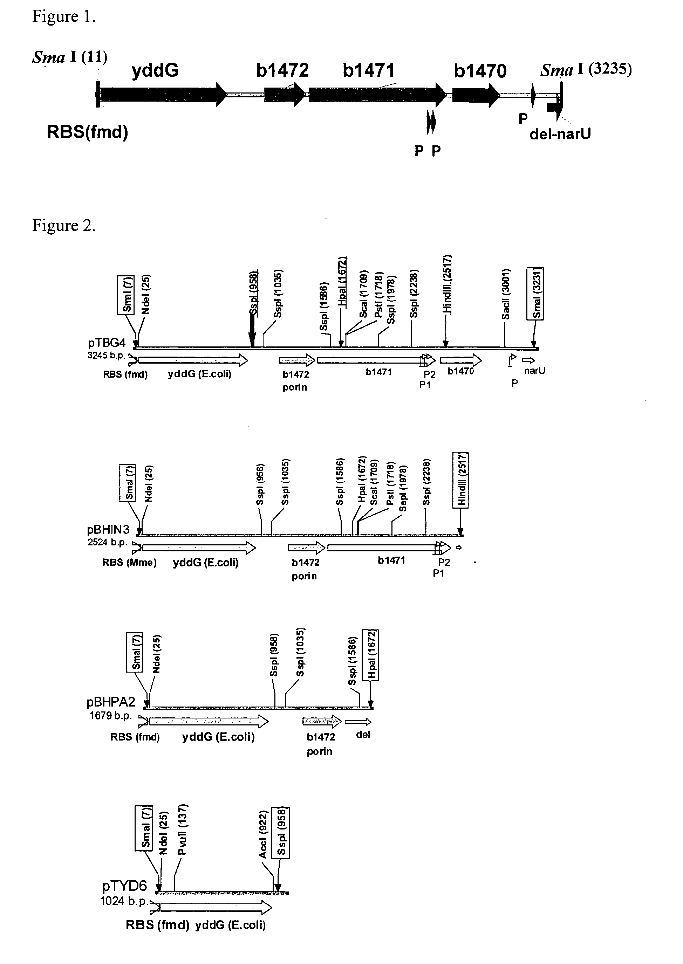

Genes located around the *yddG* gene in the *E. coli* genome were analyzed and it was found that several downstream genes encoded proteins homologous to outer membrane proteins (porins) in other bacteria. This group of *E*. *coli* genes, *yddG, yddL, yddK* and *yddJ* (Fig. 1), was amplified by PCR, cloned into pTACTER2 under the control of the P_{tac} promoter and RBS of formamidase (*fmd*) gene, and transferred into *M. methylotrophus* strain AS1.

For that purpose, the group of genes, *yddG, yddL, yddK* and *yddJ,* was amplified by PCR using primer P1 (SEQ ID NO: 9) and P3 (SEQ ID NO: 11). Primer P3 contains *Sma*I restriction site

The resulting amplified DNA fragment was treated with *Sma*I restictase and ligated under the control of the P_{tac} promoter and RBS of *fmd* gene into pTACTER2 vector which had been previously treated with the same restricase. The resulting plasmid was designated as pTBG4 (pAYCTER3-P_{tac}^{Eco}-RBS^{Mme}-yddG^{Eco}-yddL^{Eco} -yddG^{Eco} -yddJ^{Eco}). Then, the resulting pTBG4 plasmid was mobilized from *E. coli* strain S17-1 ((McPheat, W.L., FEMS Microbiology Letters, 41, 185-188 (1987)) into *M. methylotrophus* strain AS1.

The wild-type *M. methylotrophus* strain AS1 is sensitive to high concentrations of L-phenylalanine and did not grow in medium containing 1 g/L or more of L-phenylalanine. The L-phenylalanine inhibits a single DAHP synthase. This feature helps to elucidate the *E. coli* genes which encode the complete set of proteins for the phenylalanine efflux pump machinery functioning in the *M. methylotrophus* strain. The active *E. coli* L-phenylalaine efflux machinery exports L-phenylalanine from *M. methylotrophus,* reduces inner concentration of L-phenylalanine and allows bacterium to grow on medium containing L-phenylalanine in high concentration.

Several strains were tested for resistance to L-phenylalanine. These are *M. methylotrophus* strain AS1/pTBG4 (pAYCTER3-P_{tac}^{Eco}-RBS^{Mme}-yddG^{Eco}-yddL^{Eco}-yddK^{Eco}-yddJ^{Eco}) (clones No.7, No.8, No.9); AS1/pTYD6 (pAYCTER3-P_{tac}^{Eco}-_{RBS}^{Mme}-yddG^{Eco}); AS1/pAYCTER3. The phenylalanine sensitivity/resistance experiment was performed under a standard scheme. The strains were grown overnight in liquid medium, then diluted to 10⁵ cells/ml and plated on 121-medium agar plates containing 1% methanol, 100 mg/L ampicillin, and different concentrations of L-phenylalanine (g/L). The colony growth was analyzed after 72 hours. Data from the experiment are presented in Table 2.

It was determined that only the *M. methylotrophus* AS1/pTBG4 strain carrying the cloned four yddG-yddL-yddK -yddJ genes from *E. coli* can grow in a medium containing a high concentration of phenylalanine, such as 5 g/L, 2.5 g/L or 1.2 g/L.

The *M. methylotrophus* AS1/pTYD6 strain carrying a single *yddG* gene did not grow in a medium containing a high concentration of phenylalanine (1.2, 2.5, 5 g/L) similar to the control strain AS1/pAYCTER3.

Moreover, clone No.8 of *M. methylotrophus* AS1/pTBG4 strain grew poorly (leaky growth) in medium without L-phenylalanine. This can mean that the active *E. coli* phenylalanine efflux system (yddG-yddL-yddK -yddJ) really functions in the *M. methylotrophus* strain AS1 and generates leaky auxotrophy if grown in medium without L-phenylalanine.

Production of L-phenylalanine by strain AS1 and three clones of strain AS1/pTBG4 were tested by fermenting in tubes in 121 medium with 2% methanol (conditions for fermentation and method for measurement of L-phenylalanine concentration see below in the Reference example). Data from the fermentation are presented in Table 2. It can be seen that the wild-type *M. methylotrophus* strain AS1 carrying the plasmid with an active phenylalanine efflux system secreted between 0.06-0.11 g/L of L-phenylalanine into the medium.

To support the statement that all four *E. coli* genes are necessary for normal functioning of the phenylalanine efflux system in *M. methylotrophus,* two pTBG4 deletion derivatives carrying two and three Phe-efflux genes were constructed using suitable restrictases (Fig. 2) and resistance of strains AS1 carrying such derivatives to L-phenylalanine and production of L-phenylalanine by such strains were tested.

The HpaI-SmaI deletion derivative of pTBG4 plasmid, pBHA2 (pAYCTER3-P_{tac}-RBS^{fmd}-yddG^{Eco}-yddL^{Eco}), had only two *E. coli* genes and did not impart resistance to L-phenylalanine. The strain AS1 carrying that derivative did not grow in medium containing 3 g/L L-phenylalanine and did not secrete any L-phenylalanine to the medium. This means that the L-phenylalanine efflux machinery was not complete.

The HindIII-deletion derivative of pTBG4 plasmid, pBHIN3 (pAYCTER3-P_{tac}-RBS^{fmd}-yddG^{Eco}-yddL^{Eco}-yddK^{Eco}), had three *E. coli* genes. The strain AS1 carrying that derivative also did not grow in medium containing 3 g/L L-phenylalanine and did not secrete any L-phenylalanine into the medium. This result means that the all four *E. coli* genes, *yddG, yddL, yddK* and *yddJ,* are necessary for secretion of L-phenylalanine from the *M. methylotrophus* strains.

### Example 2. Fermentation procedure for M. methylotrophus strains and measurement of L-phenylalanine concentration.

### 1. Medium composition and procedure for test tube and flask cultivation

The cultivation of *M*. *methylotrophus* strains was carried out at 30 °C in 5 ml of 121 (Fe2, Fe3) medium containing 2% methanol and 3% calcium carbonate in tubes (18 mm diameter) 48-72 hours at 240 rpm on shaker.

The composition of 121(Fe2, Fe3) medium (1 liter):

| | |
|---|---|
| K₂HPO₄ x 3H₂0 | 1.57 g |
| KH₂PO₄ | 0.62 g |
| (NH₄)₂SO₄ | 3 g |
| NaCl | 0.1 g |
| MgSO₄ x 7H₂O | 0.2 g |
| CaCl₂ | 0.025 g |
| EDTA-Na₂ | 5 mg |
| FeSO₄ x 7H₂0 | 3 mg |
| FeCl₃ x 6H₂0 | 10 mg |
| MnSO₄ x 5H₂0 | 0.01 mg |
| ZnSO₄ x 7H₂0 | 0.07 mg |
| Na₂MoO₄ x 2H₂0 | 0.01 mg |
| H₃BO₃ | 0.01 mg |
| CoCl₂ x 6H₂0 | 0.005 mg |
| CuSO4 x 5H₂0 | 0.005 mg |
| Methanol | 20 ml (filter sterilized) |
| CaCO₃ | 30 g |

| | |
|---|---|
| pH 7.0. Initial volume of medium in fermentation tubes: 5 ml. | |

Seed culture was grown in 5 ml of the same liquid medium without CaCO₃ in tubes 24 hours on shaker at 240 rpm at 30°C. Inoculums size -10%. Temperature was maintained at 30 °C. Agitation - 240 rpm on shaker.

### 2. Measurement of L-phenylalanine concentration.

The L-phenylalanine concentration was measured by a fluorometric method described by, for example, McCaman, M.W. and Robins, E. (J. Lab. Clin. Med., 59, No. 5, 885-890 (1962)). Phenylalanine reacts with ninhydrin in the presence of copper ions to form a highly fluorescent complex. The fluorescence is greatly enhanced by inclusion of L-Leu-L-Ala dipeptide in the reaction mixture. The intensity of fluorescence measured in a flourometer is proportional to the phenylalanine concentration.

While the invention has been described in detail with reference to preferred embodiments thereof, it will be apparent to one skilled in the art that various changes can be made, and equivalents employed, without departing from the scope of the invention. All the cited references herein are incorporated as a part of this application by reference.

**Table 1.**

| Strain | Cloned gene | Phe, g/L |
|---|---|---|
| AS1 | - | 0.02 |
| AS1 / pTYD6 | P_{tac}^{Eco}-RBS^{Mme}-yddG^{Eco} | 0.02 |

**Table 2**

| Strain and cloned genes | Growth in the presence of L-phenylalanine, g/l | | | | | | |
|---|---|---|---|---|---|---|---|
| | 0 | 0.3 | 0.6 | 1.2 | 2.5 | 5 | 10 |
| AS1/pAYCTER3 | + | + | + | - | - | - | - |
| AS1/pTYD6 (yddG) | + | + | + | - | - | - | - |
| AS1/pTBG4 No. 7 (yddG+b1472+b1471+b1470) | + | + | + | + | ++ | ++ | - |
| AS1/pTBG4No.8 (yddG+b1472+b1471+b1470) | +/- | +/- | + | + | ++ | ++ | - |
| AS1/pTBG4 No. 9 (yddG+b1472+b1471+b1470) | + | + | + | + | + | + | - |

| | | | | | | | |
|---|---|---|---|---|---|---|---|
| +/- is a leaky growth mini colony; + is normal growth; ++ is good growth | | | | | | | |

**Table 3**

| Strain | Cloned genes | Phe, g/L |
|---|---|---|
| AS1 | - | 0.03 |
| AS1/pAYCTER3 | - | 0.05 |
| AS1/pTBG4 No. 7 | P_{tac}-RBS^{fmd}-yddG+yddL+yddK+yddJ | 0.06 |
| AS1/pTBG4 No. 9 | P_{tac}-RBS^{fmd}-yddG+yddL+yddK+yddJ | 0.09 |
| AS1/pTBG4 No.8 (leaky) | P_{tac}-RBS^{fmd}-yddG+yddL+yddK+yddJ | 0.11 |

## Claims

1. A bacterium belonging to the genus *Methylophilus* which has an ability to produce aromatic L-amino acids, wherein said bacterium has been modified so that the modified bacterium contains an increased amount of at least one protein involved in aromatic L-amino acid efflux as compared with the unmodified bacterium, and the modified bacterium is able to produce and cause accumulation of at least one aromatic L-amino acid in a culture medium in an amount higher than the unmodified bacterium.

2. The bacterium according to claim 1 which is able to produce and accumulate the at least one L-amino acid in a culture medium in an amount of not less than 0.1 g/l.

3. The bacterium according to claim 1 or 2, wherein the gene coding for said at least one protein is obtained from an *Escherichia* bacterium.

4. The bacterium according to claim 3, wherein said gene is selected from the group consisting of *yddG, yddL, yddK and yddJ.*

5. The bacterium according to any one of the preceding claims, wherein the amount of the amino acid is increased by modifying an expression control sequence which controls the expression of the gene coding for said protein.

6. The bacterium according to any one of the preceding claims, wherein said amount of the protein is increased by increasing the copy number of the gene(s) coding for said protein (s) .

7. The bacterium according to any one of the preceding claims, wherein said aromatic L-amino acid is selected from the group consisting of L-phenylalanine, L-tyrosine, and L-tryptophan.

8. The bacterium according to claim 7, wherein said L-amino acid is L-phenylalanine.

9. The bacterium according to any one of the preceding claims, wherein said bacterium is *Methylophilus methylotrophus.*

10. A method for producing an aromatic L-amino acid comprising cultivating the bacterium according to any one of the preceding claims in a medium.

11. The method of claim 10, wherein said medium contains methanol as a major carbon source.


### Annex to the application documents - subsequently filed sequences listing

SEQUENCE LISTING

<110> Ajinomoto-Genetika Research Institute

<120> METHOD FOR PRODUCING AROMATIC L-AMINO ACID USING BACTERIUM BELONGING
TO THE GENUS METHYLOPHILUS

<130> yddG-Mme

<160> 11

<170> PatentIn version 3.1

<210> 1

<211> 882

<212> DNA

<213> Escherichia coli

<220>

<221> CDS

<222> (1)..(882)

<223>

<400> 1
atg aca cga caa aaa gca acg ctc ata ggg ctg ata gcg atc gtc ctg 48
Met Thr Arg Gln Lys Ala Thr Leu Ile Gly Leu Ile Ala Ile Val Leu
1               5                   10                  15

tgg agc acg atg gta gga ttg att cgc ggt gtc agt gag ggg ctc ggc 96
Trp Ser Thr Met Val Gly Leu Ile Arg Gly Val Ser Glu Gly Leu Gly
            20                  25                  30

ccg gtc ggc ggc gca gct gct atc tat tca tta agc ggg ctg ctg tta 144
Pro Val Gly Gly Ala Ala Ala Ile Tyr Ser Leu Ser Gly Leu Leu Leu
        35                  40                  45

atc ttc acg gtt gga ttt ccg cgt att cgg caa atc ccg aaa ggc tat 192
Ile Phe Thr Val Gly Phe Pro Arg Ile Arg Gln Ile Pro Lys Gly Tyr
    50                  55                  60

tta ctc gcc ggg agt ctg tta ttc gtc agc tat gaa atc tgt ctg gcg 240
Leu Leu Ala Gly Ser Leu Leu Phe Val Ser Tyr Glu Ile Cys Leu Ala

65                  70                  75                  80

ctt tcc tta ggg tat gcg gcg acc cat cat cag gcg att gaa gtg ggt      288
Leu Ser Leu Gly Tyr Ala Ala Thr His His Gln Ala Ile Glu Val Gly
                85                  90                  95

atg gtg aac tat ctg tgg ccc agc ctg aca att ctc ttt gcc att ctg      336
Met Val Asn Tyr Leu Trp Pro Ser Leu Thr Ile Leu Phe Ala Ile Leu
            100                 105                 110

ttt aat ggt cag aaa acc aac tgg ttg att gta cct gga tta tta tta      384
Phe Asn Gly Gln Lys Thr Asn Trp Leu Ile Val Pro Gly Leu Leu Leu
        115                 120                 125

gcc ctc gtc ggc gtc tgt tgg gtg tta ggc ggt gac aat ggg tta cat      432
Ala Leu Val Gly Val Cys Trp Val Leu Gly Gly Asp Asn Gly Leu His
    130                 135                 140

tat gat gaa atc atc aat aat atc acc acc agc cca ttg agt tat ttc      480
Tyr Asp Glu Ile Ile Asn Asn Ile Thr Thr Ser Pro Leu Ser Tyr Phe
145                 150                 155                 160

ctg gcg ttc att ggt gcg ttt atc tgg gca gcc tat tgc aca gta acg      528
Leu Ala Phe Ile Gly Ala Phe Ile Trp Ala Ala Tyr Cys Thr Val Thr
                165                 170                 175

aat aaa tac gca cgc gga ttt aat gga att acc gtt ttt gtc ctg cta      576
Asn Lys Tyr Ala Arg Gly Phe Asn Gly Ile Thr Val Phe Val Leu Leu
            180                 185                 190

acg gga gca agt ctg tgg gtt tac tat ttt ctt acg cca caa cca gaa      624
Thr Gly Ala Ser Leu Trp Val Tyr Tyr Phe Leu Thr Pro Gln Pro Glu
        195                 200                 205

atg ata ttt agc acg ccc gtc atg att aaa ctc atc tct gcg gca ttt      672
Met Ile Phe Ser Thr Pro Val Met Ile Lys Leu Ile Ser Ala Ala Phe
    210                 215                 220

acc tta gga ttt gct tat gct gca tgg aat gtc ggt ata ttg cat ggc      720
Thr Leu Gly Phe Ala Tyr Ala Ala Trp Asn Val Gly Ile Leu His Gly
225                 230                 235                 240

aat gtc acc att atg gcg gta ggt tcg tat ttt acg cct gta ctt tcc      768
Asn Val Thr Ile Met Ala Val Gly Ser Tyr Phe Thr Pro Val Leu Ser
                245                 250                 255

tca gcg ctt gca gcc gtg ctg ctc agc gcc ccg ctg tcg ttc tcg ttc      816
Ser Ala Leu Ala Ala Val Leu Leu Ser Ala Pro Leu Ser Phe Ser Phe
            260                 265                 270

tgg caa ggc gcg ctg atg gtc tgc ggc ggt tcc ctg ctc tgc tgg ctg      864
Trp Gln Gly Ala Leu Met Val Cys Gly Gly Ser Leu Leu Cys Trp Leu
        275                 280                 285

gcg aca cgt cgt ggt taa                                              882
Ala Thr Arg Arg Gly
    290

<210>  2

<211>  293

<212>  PRT

<213>  Escherichia coli

<400>  2

Met Thr Arg Gln Lys Ala Thr Leu Ile Gly Leu Ile Ala Ile Val Leu
1               5                   10                  15

Trp Ser Thr Met Val Gly Leu Ile Arg Gly Val Ser Glu Gly Leu Gly
            20                  25                  30

Pro Val Gly Gly Ala Ala Ala Ile Tyr Ser Leu Ser Gly Leu Leu Leu
        35                  40                  45

Ile Phe Thr Val Gly Phe Pro Arg Ile Arg Gln Ile Pro Lys Gly Tyr
    50                  55                  60

Leu Leu Ala Gly Ser Leu Leu Phe Val Ser Tyr Glu Ile Cys Leu Ala
65                  70                  75                  80

Leu Ser Leu Gly Tyr Ala Ala Thr His His Gln Ala Ile Glu Val Gly
                85                  90                  95

Met Val Asn Tyr Leu Trp Pro Ser Leu Thr Ile Leu Phe Ala Ile Leu
            100                 105                 110

Phe Asn Gly Gln Lys Thr Asn Trp Leu Ile Val Pro Gly Leu Leu Leu
        115                 120                 125

Ala Leu Val Gly Val Cys Trp Val Leu Gly Gly Asp Asn Gly Leu His
    130                 135                 140

Tyr Asp Glu Ile Ile Asn Asn Ile Thr Thr Ser Pro Leu Ser Tyr Phe
145                 150                 155                 160

Leu Ala Phe Ile Gly Ala Phe Ile Trp Ala Ala Tyr Cys Thr Val Thr
                165                 170                 175

Asn Lys Tyr Ala Arg Gly Phe Asn Gly Ile Thr Val Phe Val Leu Leu
            180                 185                 190

Thr Gly Ala Ser Leu Trp Val Tyr Tyr Phe Leu Thr Pro Gln Pro Glu
        195                 200                 205

Met Ile Phe Ser Thr Pro Val Met Ile Lys Leu Ile Ser Ala Ala Phe
    210                 215                 220

Thr Leu Gly Phe Ala Tyr Ala Ala Trp Asn Val Gly Ile Leu His Gly
225                 230                 235                 240

Asn Val Thr Ile Met Ala Val Gly Ser Tyr Phe Thr Pro Val Leu Ser
                245                 250                 255

Ser Ala Leu Ala Ala Val Leu Leu Ser Ala Pro Leu Ser Phe Ser Phe
            260                 265                 270

Trp Gln Gly Ala Leu Met Val Cys Gly Gly Ser Leu Leu Cys Trp Leu
        275                 280                 285

Ala Thr Arg Arg Gly
    290

<210>  3

<211>  291

<212>  DNA

<213>  Escherichia coli

<220>

<221>  CDS

<222>  (1)..(291)

<223>

<400>  3
atg aaa tta aaa ata gtt gcg gtg gtt gta act ggt ttg tta gct gcg       48
Met Lys Leu Lys Ile Val Ala Val Val Val Thr Gly Leu Leu Ala Ala
1               5                   10                  15

aac gta gca cac gct gcc gaa gtc tat aac aag gat ggt aat aaa ctc       96
Asn Val Ala His Ala Ala Glu Val Tyr Asn Lys Asp Gly Asn Lys Leu
            20                  25                  30

gac ctt tat ggc aag gtt acc gct cta cgt tat ttt act gat gat aag      144
Asp Leu Tyr Gly Lys Val Thr Ala Leu Arg Tyr Phe Thr Asp Asp Lys
        35                  40                  45

cgt gac gat ggt gat aaa act tat gcc cgt ctc ggc ttt aaa gga gaa      192
Arg Asp Asp Gly Asp Lys Thr Tyr Ala Arg Leu Gly Phe Lys Gly Glu
    50                  55                  60

acg caa atc aat gat caa atg att ggt ttt ggt cac tgg gaa tat gat      240
Thr Gln Ile Asn Asp Gln Met Ile Gly Phe Gly His Trp Glu Tyr Asp
65                  70                  75                  80

ttt aaa ggc tat aac gat gaa gcc aac ggc tcg cgc ggc aag aat ctc      288
Phe Lys Gly Tyr Asn Asp Glu Ala Asn Gly Ser Arg Gly Lys Asn Leu
                85                  90                  95

tga                                                                 291

<210>  4

<211>  96

<212>  PRT

<213>  Escherichia coli

<400>  4

Met Lys Leu Lys Ile Val Ala Val Val Val Thr Gly Leu Leu Ala Ala
1               5                   10                  15

Asn Val Ala His Ala Ala Glu Val Tyr Asn Lys Asp Gly Asn Lys Leu
            20                  25                  30

Asp Leu Tyr Gly Lys Val Thr Ala Leu Arg Tyr Phe Thr Asp Asp Lys
        35                  40                  45

Arg Asp Asp Gly Asp Lys Thr Tyr Ala Arg Leu Gly Phe Lys Gly Glu
    50                  55                  60

Thr Gln Ile Asn Asp Gln Met Ile Gly Phe Gly His Trp Glu Tyr Asp
65                  70                  75                  80

Phe Lys Gly Tyr Asn Asp Glu Ala Asn Gly Ser Arg Gly Lys Asn Leu
                85                  90                  95

<210>  5

<211>  957

<212>  DNA

<213>  Escherichia coli

<220>

<221>  CDS

<222>  (1)..(957)

<223>

<400>  5
atg atc act gac ctt ata tta cac aat cat ccc agg atg aaa aca atc      48
act tta aac gac aac cat att gca cat tta aac gcc aaa aac act aca 96
aaa ctg gaa tat tta aac tta agc aat aac aat tta ctg cca acc aat 144
gac att gat caa cta ata tca tca aaa cat ctt tgg cat gta tta gtt 192
aac ggc atc aac aat gat cca ctt gca caa atg cag tac tgg act gca 240
gta aga aat ata att gat gac act aat gaa gtg acc att gat tta tca 288
gga ctt aat tta acc act caa cca cca ggg ctg caa aac ttc aca tct 336
atc aat ctt gat aat aac caa ttc aca cat ttt gat gca acc aac tac 384
gat aga ctc gta aag cta agt ctg aat agt aat gct ctt gag tca ata 432
aat ttt cct caa ggc aga aat gta agt att aca cat ata tct atg aat 480
aat aat gct ctc aga aat att gat ata gat agg ctt tca tca gtt act 528
tat ttt agt gcg gca cat aat caa cta gag ttt gtg caa tta gaa tct 576
tgc gaa tgg ctg caa tac ctg aat ctc agc cat aat caa tta act gat 624
att gtt gca ggt aat aaa aat gaa ctc tta ctg ctg gat cta tcc cat 672
aat aaa cta aca agt tta cac aat gac tta ttt ccc aac ttg aat acg 720
tta ctt att aac aac aat ttg ctt tct gaa att aaa ata ttc tat agc 768
aac ttc tgc aat gtt cag aca tta aac gct gct aac aac cag ttg aaa 816
tat ata aat ctt gat ttc ctg act tat ctt cca tct atc aaa agt tta 864
aga ctg gac aat aat aaa ata acc cac att gat act aat aat aca tcc 912
gat att gga act tta ttc ccc ata ata aaa cag agc aaa act taa 957

<210> 6

<211> 318

<212> PRT

<213> Escherichia coli

<400> 6

<210>  7

<211>  336

<212>  DNA

<213>  Escherichia coli

<220>

<221>  CDS

<222>  (1)..(336)

<223>

<400>  7
atg cag ctc atg tta ttt aat ttg ttt tcc cca gca ctt aag ctt aat        48

Met Gln Leu Met Leu Phe Asn Leu Phe Ser Pro Ala Leu Lys Leu Asn
1               5                   10                  15

act ggc ctg gca att ctt tcg cct ggt gca ttt gaa gtt cac tct gac        96
Thr Gly Leu Ala Ile Leu Ser Pro Gly Ala Phe Glu Val His Ser Asp
            20                  25                  30

gga ata gat gtg gat aac gaa ttg ttt cac tat cct att aaa aaa gca        144
Gly Ile Asp Val Asp Asn Glu Leu Phe His Tyr Pro Ile Lys Lys Ala
        35                  40                  45

tat acc cca tat aat ata cac act tac aag aca gag gaa gtt gta aac        192
Tyr Thr Pro Tyr Asn Ile His Thr Tyr Lys Thr Glu Glu Val Val Asn
    50                  55                  60

cag atg aat ata aaa gtt aaa aac atg acc tta gat gaa ata aac aat        240
Gln Met Asn Ile Lys Val Lys Asn Met Thr Leu Asp Glu Ile Asn Asn
65                  70                  75                  80

act tac tgt aat aac gat tat tac aat cag gca ata aga gag gaa ccg        288
Thr Tyr Cys Asn Asn Asp Tyr Tyr Asn Gln Ala Ile Arg Glu Glu Pro
                85                  90                  95

ata gac ctt ctg gac aga tcg ttt tcc tcc agt tca tgg cct ttt tag        336
Ile Asp Leu Leu Asp Arg Ser Phe Ser Ser Ser Ser Trp Pro Phe
            100                 105                 110

<210>  8

<211>  111

<212>  PRT

<213>  Escherichia coli

<400>  8

Met Gln Leu Met Leu Phe Asn Leu Phe Ser Pro Ala Leu Lys Leu Asn
1               5                   10                  15

Thr Gly Leu Ala Ile Leu Ser Pro Gly Ala Phe Glu Val His Ser Asp
            20                  25                  30

Gly Ile Asp Val Asp Asn Glu Leu Phe His Tyr Pro Ile Lys Lys Ala
        35                  40                  45

Tyr Thr Pro Tyr Asn Ile His Thr Tyr Lys Thr Glu Glu Val Val Asn
    50                  55                  60

Gln Met Asn Ile Lys Val Lys Asn Met Thr Leu Asp Glu Ile Asn Asn
65                  70                  75                  80

Thr Tyr Cys Asn Asn Asp Tyr Tyr Asn Gln Ala Ile Arg Glu Glu Pro
                85                  90                  95

Ile Asp Leu Leu Asp Arg Ser Phe Ser Ser Ser Ser Trp Pro Phe
            100                 105                 110

<210>  9

<211>  52

<212>  DNA

<213>  Artificial sequence: primer

<400>  9
ttaacggccc gggaaggaga atatacatat gacacgacaa aaagcaacgc tc          52

<210>  10

<211>  27

<212>  DNA

<213>  Artificial sequence: primer

<400>  10
tctgatcggt accgaaatga gatataa                                      27

<210>  11

<211>  37

<212>  DNA

<213>  Artificial sequence: primer

<400>  11
agagatttcc cgggcaatat gtttcctttt attttcc                           37

SEQUENCE LISTING

<110>  Ajinomoto-Genetika Research Institute

<120>  METHOD FOR PRODUCING AROMATIC L-AMINO ACID USING BACTERIUM BELONGING TO
THE GENUS METHYLOPHILUS

<130>  yddG-Mme
<140>  EP06025149.3
<141>  2006-12-05

<160>  11

<170>  PatentIn version 3.1

<210>  1
<211>  882
<212>  DNA
<213>  Escherichia coli

<220>
<221>  CDS
<222>  (1)..(882)

<400>  1
atg aca cga caa aaa gca acg ctc ata ggg ctg ata gcg atc gtc ctg       48
Met Thr Arg Gln Lys Ala Thr Leu Ile Gly Leu Ile Ala Ile Val Leu
1               5                   10                  15

tgg agc acg atg gta gga ttg att cgc ggt gtc agt gag ggg ctc ggc       96
Trp Ser Thr Met Val Gly Leu Ile Arg Gly Val Ser Glu Gly Leu Gly
            20                  25                  30

ccg gtc ggc ggc gca gct gct atc tat tca tta agc ggg ctg ctg tta      144
Pro Val Gly Gly Ala Ala Ala Ile Tyr Ser Leu Ser Gly Leu Leu Leu
        35                  40                  45

atc ttc acg gtt gga ttt ccg cgt att cgg caa atc ccg aaa ggc tat      192
Ile Phe Thr Val Gly Phe Pro Arg Ile Arg Gln Ile Pro Lys Gly Tyr
    50                  55                  60

tta ctc gcc ggg agt ctg tta ttc gtc agc tat gaa atc tgt ctg gcg      240
Leu Leu Ala Gly Ser Leu Leu Phe Val Ser Tyr Glu Ile Cys Leu Ala
65                  70                  75                  80

ctt tcc tta ggg tat gcg gcg acc cat cat cag gcg att gaa gtg ggt      288
Leu Ser Leu Gly Tyr Ala Ala Thr His His Gln Ala Ile Glu Val Gly
                85                  90                  95

atg gtg aac tat ctg tgg ccc agc ctg aca att ctc ttt gcc att ctg      336
Met Val Asn Tyr Leu Trp Pro Ser Leu Thr Ile Leu Phe Ala Ile Leu
            100                 105                 110

ttt aat ggt cag aaa acc aac tgg ttg att gta cct gga tta tta tta      384
Phe Asn Gly Gln Lys Thr Asn Trp Leu Ile Val Pro Gly Leu Leu Leu
        115                 120                 125

gcc ctc gtc ggc gtc tgt tgg gtg tta ggc ggt gac aat ggg tta cat      432
Ala Leu Val Gly Val Cys Trp Val Leu Gly Gly Asp Asn Gly Leu His
    130                 135                 140

tat gat gaa atc atc aat aat atc acc acc agc cca ttg agt tat ttc 480
Tyr Asp Glu Ile Ile Asn Asn Ile Thr Thr Ser Pro Leu Ser Tyr Phe
145                 150                 155                 160

ctg gcg ttc att ggt gcg ttt atc tgg gca gcc tat tgc aca gta acg 528
Leu Ala Phe Ile Gly Ala Phe Ile Trp Ala Ala Tyr Cys Thr Val Thr
                165                 170                 175

aat aaa tac gca cgc gga ttt aat gga att acc gtt ttt gtc ctg cta 576
Asn Lys Tyr Ala Arg Gly Phe Asn Gly Ile Thr Val Phe Val Leu Leu
            180                 185                 190

acg gga gca agt ctg tgg gtt tac tat ttt ctt acg cca caa cca gaa 624
Thr Gly Ala Ser Leu Trp Val Tyr Tyr Phe Leu Thr Pro Gln Pro Glu
        195                 200                 205

atg ata ttt agc acg ccc gtc atg att aaa ctc atc tct gcg gca ttt 672
Met Ile Phe Ser Thr Pro Val Met Ile Lys Leu Ile Ser Ala Ala Phe
    210                 215                 220

acc tta gga ttt gct tat gct gca tgg aat gtc ggt ata ttg cat ggc 720
Thr Leu Gly Phe Ala Tyr Ala Ala Trp Asn Val Gly Ile Leu His Gly
225                 230                 235                 240

aat gtc acc att atg gcg gta ggt tcg tat ttt acg cct gta ctt tcc 768
Asn Val Thr Ile Met Ala Val Gly Ser Tyr Phe Thr Pro Val Leu Ser
                245                 250                 255

tca gcg ctt gca gcc gtg ctg ctc agc gcc ccg ctg tcg ttc tcg ttc 816
Ser Ala Leu Ala Ala Val Leu Leu Ser Ala Pro Leu Ser Phe Ser Phe
            260                 265                 270

tgg caa ggc gcg ctg atg gtc tgc ggc ggt tcc ctg ctc tgc tgg ctg 864
Trp Gln Gly Ala Leu Met Val Cys Gly Gly Ser Leu Leu Cys Trp Leu
        275                 280                 285

gcg aca cgt cgt ggt taa 882
Ala Thr Arg Arg Gly
    290

<210> 2
<211> 293
<212> PRT
<213> Escherichia coli

<400> 2

Met Thr Arg Gln Lys Ala Thr Leu Ile Gly Leu Ile Ala Ile Val Leu
1               5                   10                  15

Trp Ser Thr Met Val Gly Leu Ile Arg Gly Val Ser Glu Gly Leu Gly
            20                  25                  30

Pro Val Gly Gly Ala Ala Ala Ile Tyr Ser Leu Ser Gly Leu Leu Leu
        35                  40                  45

Ile Phe Thr Val Gly Phe Pro Arg Ile Arg Gln Ile Pro Lys Gly Tyr

50                  55                  60

Leu Leu Ala Gly Ser Leu Leu Phe Val Ser Tyr Glu Ile Cys Leu Ala
65                  70                  75                  80

Leu Ser Leu Gly Tyr Ala Ala Thr His His Gln Ala Ile Glu Val Gly
                85                  90                  95

Met Val Asn Tyr Leu Trp Pro Ser Leu Thr Ile Leu Phe Ala Ile Leu
            100                 105                 110

Phe Asn Gly Gln Lys Thr Asn Trp Leu Ile Val Pro Gly Leu Leu Leu
        115                 120                 125

Ala Leu Val Gly Val Cys Trp Val Leu Gly Gly Asp Asn Gly Leu His
    130                 135                 140

Tyr Asp Glu Ile Ile Asn Asn Ile Thr Thr Ser Pro Leu Ser Tyr Phe
145                 150                 155                 160

Leu Ala Phe Ile Gly Ala Phe Ile Trp Ala Ala Tyr Cys Thr Val Thr
                165                 170                 175

Asn Lys Tyr Ala Arg Gly Phe Asn Gly Ile Thr Val Phe Val Leu Leu
            180                 185                 190

Thr Gly Ala Ser Leu Trp Val Tyr Tyr Phe Leu Thr Pro Gln Pro Glu
        195                 200                 205

Met Ile Phe Ser Thr Pro Val Met Ile Lys Leu Ile Ser Ala Ala Phe
    210                 215                 220

Thr Leu Gly Phe Ala Tyr Ala Ala Trp Asn Val Gly Ile Leu His Gly
225                 230                 235                 240

Asn Val Thr Ile Met Ala Val Gly Ser Tyr Phe Thr Pro Val Leu Ser
                245                 250                 255

Ser Ala Leu Ala Ala Val Leu Leu Ser Ala Pro Leu Ser Phe Ser Phe
            260                 265                 270

Trp Gln Gly Ala Leu Met Val Cys Gly Gly Ser Leu Leu Cys Trp Leu
        275                 280                 285

Ala Thr Arg Arg Gly
    290

<210>  3
<211>  291
<212>  DNA
<213>  Escherichia coli

<220>
<221>  CDS
<222>  (1)..(291)

<400>  3
atg aaa tta aaa ata gtt gcg gtg gtt gta act ggt ttg tta gct gcg      48
Met Lys Leu Lys Ile Val Ala Val Val Val Thr Gly Leu Leu Ala Ala
1               5                   10                  15

aac gta gca cac gct gcc gaa gtc tat aac aag gat ggt aat aaa ctc      96
Asn Val Ala His Ala Ala Glu Val Tyr Asn Lys Asp Gly Asn Lys Leu
            20                  25                  30

gac ctt tat ggc aag gtt acc gct cta cgt tat ttt act gat gat aag      144
Asp Leu Tyr Gly Lys Val Thr Ala Leu Arg Tyr Phe Thr Asp Asp Lys
        35                  40                  45

cgt gac gat ggt gat aaa act tat gcc cgt ctc ggc ttt aaa gga gaa      192
Arg Asp Asp Gly Asp Lys Thr Tyr Ala Arg Leu Gly Phe Lys Gly Glu
    50                  55                  60

acg caa atc aat gat caa atg att ggt ttt ggt cac tgg gaa tat gat      240
Thr Gln Ile Asn Asp Gln Met Ile Gly Phe Gly His Trp Glu Tyr Asp
65                  70                  75                  80

ttt aaa ggc tat aac gat gaa gcc aac ggc tcg cgc ggc aag aat ctc      288
Phe Lys Gly Tyr Asn Asp Glu Ala Asn Gly Ser Arg Gly Lys Asn Leu
                85                  90                  95

tga                                                                  291


<210>  4
<211>  96
<212>  PRT
<213>  Escherichia coli

<400>  4

Met Lys Leu Lys Ile Val Ala Val Val Val Thr Gly Leu Leu Ala Ala
1               5                   10                  15


Asn Val Ala His Ala Ala Glu Val Tyr Asn Lys Asp Gly Asn Lys Leu
            20                  25                  30


Asp Leu Tyr Gly Lys Val Thr Ala Leu Arg Tyr Phe Thr Asp Asp Lys
        35                  40                  45


Arg Asp Asp Gly Asp Lys Thr Tyr Ala Arg Leu Gly Phe Lys Gly Glu
    50                  55                  60

Thr Gln Ile Asn Asp Gln Met Ile Gly Phe Gly His Trp Glu Tyr Asp
65                  70                  75                  80

Phe Lys Gly Tyr Asn Asp Glu Ala Asn Gly Ser Arg Gly Lys Asn Leu
                85                  90                  95

<210>  5
<211>  957
<212>  DNA
<213>  Escherichia coli

<220>
<221>  CDS
<222>  (1)..(957)

<400>  5
atg atc act gac ctt ata tta cac aat cat ccc agg atg aaa aca atc       48
act tta aac gac aac cat att gca cat tta aac gcc aaa aac act aca       96
aaa ctg gaa tat tta aac tta agc aat aac aat tta ctg cca acc aat      144
gac att gat caa cta ata tca tca aaa cat ctt tgg cat gta tta gtt      192
aac ggc atc aac aat gat cca ctt gca caa atg cag tac tgg act gca      240
gta aga aat ata att gat gac act aat gaa gtg acc att gat tta tca      288
gga ctt aat tta acc act caa cca cca ggg ctg caa aac ttc aca tct      336
atc aat ctt gat aat aac caa ttc aca cat ttt gat gca acc aac tac      384
gat aga ctc gta aag cta agt ctg aat agt aat gct ctt gag tca ata      432
aat ttt cct caa ggc aga aat gta agt att aca cat ata tct atg aat      480
aat aat gct ctc aga aat att gat ata gat agg ctt tca tca gtt act      528
tat ttt agt gcg gca cat aat caa cta gag ttt gtg caa tta gaa tct 576
tgc gaa tgg ctg caa tac ctg aat ctc agc cat aat caa tta act gat 624
att gtt gca ggt aat aaa aat gaa ctc tta ctg ctg gat cta tcc cat 672
aat aaa cta aca agt tta cac aat gac tta ttt ccc aac ttg aat acg 720
tta ctt att aac aac aat ttg ctt tct gaa att aaa ata ttc tat agc 768
aac ttc tgc aat gtt cag aca tta aac gct gct aac aac cag ttg aaa 816
tat ata aat ctt gat ttc ctg act tat ctt cca tct atc aaa agt tta 864
aga ctg gac aat aat aaa ata acc cac att gat act aat aat aca tcc 912
gat att gga act tta ttc ccc ata ata aaa cag agc aaa act taa 957

<210> 6
<211> 318
<212> PRT
<213> Escherichia coli

<400> 6

<210>  7
<211>  336
<212>  DNA
<213>  Escherichia coli

<220>
<221>  CDS
<222>  (1)..(336)

<400>  7
atg cag ctc atg tta ttt aat ttg ttt tcc cca gca ctt aag ctt aat        48
Met Gln Leu Met Leu Phe Asn Leu Phe Ser Pro Ala Leu Lys Leu Asn
1               5                   10                  15

act ggc ctg gca att ctt tcg cct ggt gca ttt gaa gtt cac tct gac        96
Thr Gly Leu Ala Ile Leu Ser Pro Gly Ala Phe Glu Val His Ser Asp
            20                  25                  30

gga ata gat gtg gat aac gaa ttg ttt cac tat cct att aaa aaa gca       144
Gly Ile Asp Val Asp Asn Glu Leu Phe His Tyr Pro Ile Lys Lys Ala
        35                  40                  45

tat acc cca tat aat ata cac act tac aag aca gag gaa gtt gta aac       192
Tyr Thr Pro Tyr Asn Ile His Thr Tyr Lys Thr Glu Glu Val Val Asn
    50                  55                  60

cag atg aat ata aaa gtt aaa aac atg acc tta gat gaa ata aac aat       240
Gln Met Asn Ile Lys Val Lys Asn Met Thr Leu Asp Glu Ile Asn Asn
65                  70                  75                  80

act tac tgt aat aac gat tat tac aat cag gca ata aga gag gaa ccg       288
Thr Tyr Cys Asn Asn Asp Tyr Tyr Asn Gln Ala Ile Arg Glu Glu Pro
                85                  90                  95

ata gac ctt ctg gac aga tcg ttt tcc tcc agt tca tgg cct ttt tag       336
Ile Asp Leu Leu Asp Arg Ser Phe Ser Ser Ser Ser Trp Pro Phe
            100                 105                 110


<210>  8
<211>  111
<212>  PRT
<213>  Escherichia coli

<400>  8

Met Gln Leu Met Leu Phe Asn Leu Phe Ser Pro Ala Leu Lys Leu Asn
1               5                   10                  15


Thr Gly Leu Ala Ile Leu Ser Pro Gly Ala Phe Glu Val His Ser Asp
            20                  25                  30


Gly Ile Asp Val Asp Asn Glu Leu Phe His Tyr Pro Ile Lys Lys Ala
        35                  40                  45


Tyr Thr Pro Tyr Asn Ile His Thr Tyr Lys Thr Glu Glu Val Val Asn

50                  55                  60

Gln Met Asn Ile Lys Val Lys Asn Met Thr Leu Asp Glu Ile Asn Asn
65                  70                  75                  80

Thr Tyr Cys Asn Asn Asp Tyr Tyr Asn Gln Ala Ile Arg Glu Glu Pro
                85                  90                  95

Ile Asp Leu Leu Asp Arg Ser Phe Ser Ser Ser Ser Trp Pro Phe
            100                 105                 110

<210>  9
<211>  52
<212>  DNA
<213>  Artificial sequence: primer

<220>
<223>  Description of the Artificial Sequence: synthetic DNA

<400>  9
ttaacggccc gggaaggaga atatacatat gacacgacaa aaagcaacgc tc          52

<210>  10
<211>  27
<212>  DNA
<213>  Artificial sequence: primer

<220>
<223>  Description of the Artificial Sequence: synthetic DNA

<400>  10
tctgatcggt accgaaatga gatataa                                      27

<210>  11
<211>  37
<212>  DNA
<213>  Artificial sequence: primer

<220>
<223>  Description of the Artificial Sequence: synthetic DNA

<400>  11
agagatttcc cgggcaatat gttttccttt attttcc                           37